# EUROPÄISCHE PATENTSCHRIFT

(11) **EP 2 539 681 B1**
(45) Veröffentlichungstag und Bekanntmachung des Hinweises auf die Patenterteilung: **01.01.2020**
(21) Anmeldenummer: 11700518.1
(22) Anmeldetag: 04.01.2011
(51) Int. Cl.: G01N 33/00, G01N 21/27, G01N 1/22, G01N 15/06, G01N 21/53, G01N 21/51

(54) **VORRICHTUNG ZUR MESSUNG EINER PARTIKELKONZENTRATION IN KRAFTFAHRZEUGABGASEN**
DEVICE FOR MEASURING A PARTICLE CONCENTRATION IN MOTOR VEHICLE EXHAUST GASES
DISPOSITIF POUR MESURER LA CONCENTRATION EN PARTICULES DANS LES GAZ D'ECHAPPEMENT DE VEHICULES A MOTEUR

(30) Priorität: 26.02.2010 DE 102010002424
(43) Veröffentlichungstag der Anmeldung: 02.01.2013
(73) Patentinhaber: Robert Bosch GmbH, 70442 Stuttgart (DE)
(72) Erfinder: STENGEL, Karl, 73779 Deizisau (DE); HAAGA, Gerhard, 73275 Ohmden (DE); NEUENDORF, Michael, 73207 Plochingen (DE); SIEG, Raymond, 73732 Esslingen (DE)
(86) Internationale Anmeldenummer: PCT/EP2011/050048
(87) Internationale Veröffentlichungsnummer: WO 2011/104043

(56) Entgegenhaltungen:
- EP-A2- 0 967 481
- AT-U2- 7 031
- DE-A1- 4 117 529
- DE-A1- 4 213 051
- US-A- 4 063 446
- ANONYMOUS: "Anforderungen für die Messung von Russpartikeln, Streulicht Photometer", INTERNET CITATION, 6. März 2009 (2009-03-06), Seiten 1-4, XP002600226, Gefunden im Internet: URL:http://www.eastsolutions.eu/html/messi gkeiten.html [gefunden am 2010-09-10]

## Beschreibung

### Stand der Technik

Die Erfindung betrifft eine Vorrichtung zur Messung einer Partikelkonzentration in Kraftfahrzeugabgasen.

Zur Messung von Partikeln in Abgasen und anderen Kolloiden wird häufig ein Streulichtverfahren eingesetzt, bei dem das zu messende Kolloid durch eine Messkammer geleitet wird. Dabei wird beispielsweise ein Laser als Lichtquelle derart eingesetzt, dass das von der Lichtquelle ausgesendete Licht von im Kolloid vorhandenen Partikeln gestreut wird. Zusätzlich sind mindestens ein, besser aber mehrere Lichtsensoren vorhanden, die das von den Partikeln gestreute Licht (Streulicht) detektieren. Die Lichtsensoren können unter verschiedenen Winkeln in Bezug zur Einstrahlrichtung des Lichts angeordnet sein.

Grundsätzlich weisen die beteiligten Komponenten wie Lichtquelle, Lichtsensoren und die elektronische Auswertungsschaltung der Sensorsignale diverse Offset- und Driftmechanismen auf, welche zeitlich invariant, in der Regel aber sehr langsam erfolgen. Diese Mechanismen umfassen beispielsweise eine Temperaturdrift der von der Lichtquelle emittierten Lichtleistung, eine Drift der Sensoren, eine Drift der Auswerteschaltung, Offsetgrößen dieser Komponenten und Alterungseffekte. Neben diesen durch die elektronischen Komponenten hervorgerufenen Messfehlern kommen bei Streulichtverfahren noch weitere mögliche Fehlerquellen hinzu. Beispiele hierfür sind Reflexionen an der Messkammergeometrie, wenn die Lichtquelle eingeschaltet ist, auch wenn sich keine Partikel in der Messkammer befinden, und andere Signalanteile durch Partikel, weil die Messkammer mit einem Medium gefüllt ist, z.B. einem Gas oder Umgebungsluft, welche(s) eine gewisse, wenn auch im Vergleich zu den Abgasen geringere Menge an Partikeln enthält.

Aus der DE 41 17529 A1 und DE 34 14542 A1 sind Vorrichtungen Zur Messung von Rußpastikeln in Verbrennungsmotoren bekannt.

Im Falle von Prüfgeräten, die in Werkstätten eingesetzt werden, ist die Umgebungsluft häufig sehr stark mit Partikeln verschmutzt und beeinflusst die Mess-genauigkeit daher merklich.

Insbesondere wenn mit dem Messgerät die Emissionen von modernen Kraftfahrzeugen mit Partikelfiltern beurteilt werden sollen, die ein sehr niedriges Emissionsniveau aufweisen, haben die beschriebenen Mechanismen einen großen Einfluss auf die Genauigkeit der Messung und Diagnose.

Es besteht somit der Bedarf, die zuvor beschriebenen Fehlerquellen zu eliminieren, um die Partikelkonzentration in Kraftfahrzeugabgasen mit hoher Genauigkeit messen zu können.

### Offenbarung der Erfindung

Es ist eine Aufgabe der Erfindung, ein Emissionsmessgerät zur Messung wenigstens einer Partikelkonzentration in Kraftfahrzeugabgasen bereitzustellen, mit dem die Partikelkonzentration mit hoher Genauigkeit bestimmbar ist.

Diese Aufgabe wird erfindungsgemäß gelöst durch eine Vorrichtung zur Messung einer Partikelkonzentration in Kraftfahrzeugabgasen nach dem unabhängigen Patentanspruch 1. Die abhängigen Patentansprüche beschreiben vorteilhafte Ausgestaltungen einer erfindungsgemäßen Vorrichtung.

Eine Vorrichtung zur Messung einer Partikelkonzentration in Kraftfahrzeugabgasen hat eine Messkammer, die mit einer zu messenden Gas-Partikel-Mischung (Kolloid) befüllbar ist und weist wenigstens eine Lichtquelle und wenigstens einen Lichtsensor auf, wobei der Lichtsensor ausgebildet ist, um von der Lichtquelle ausgestrahltes und von den in der Gas-Partikel-Mischung vorhandenen Partikeln gestreutes Licht (Streulicht) zu detektieren. Die erfindungsgemäße Vorrichtung weist auch Abgaszuführeinrichtung und eine Nullgasquelle auf. Die Abgaszuführeinrichtung ist ausgebildet, um zu messendes Abgas in die Messkammer zu leiten. Die Nullgasquelle ist ausgebildet, um der Messkammer ein partikelarmes Nullgas zuzuführen. Ein Schaltelement, das zwischen der Abgaszuführeinrichtung und der Messkammer angeordnet ist, ist geeignet, die Zufuhr von Abgas in die Messkammer wahlweise zuzulassen oder zu unterbinden.

Durch eine Vorrichtung mit einem Schaltelement, welches geeignet ist, die Zufuhr von Abgas in die Messkammer zu unterbinden, kann ein Nullabgleich einfach durchgeführt werden, ohne das dafür beispielsweise Schläuche abgezogen und/oder umgesteckt werden müssen. Durch das regelmäßige Durchführen eines Nullabgleichs wird die Genauigkeit der von der Messvorrichtung ermittelten Messergebnisse verbessert.

Auch schafft eine solche Vorrichtung die Möglichkeit, automatisch einen Nullabgleich des Messsystems durchführen zu können und so automatisch eine ausreichende Genauigkeit der Messergebnisse sicherzustellen.

In einer Ausführungsform, die nicht der Erfindung entspricht, ist ein Schaltelement vorgesehen, das zwischen der Nullgasquelle und der Messkammer angeordnet und geeignet ist, die Zufuhr von Nullgas in die Messkammer wahlweise zuzulassen oder zu unterbinden. Dadurch, dass die Zufuhr von Nullgas in die Messkammer während des Messvorgangs absperrbar ist, kann die Messung besonders effektiv und genau durchgeführt werden, da die Messergebnisse nicht durch zugemischtes Nullgas verfälscht werden.

In dieser Ausführungsform ist das Schaltelement ein Umschaltelement, welches derart ausgebildet ist, dass es die Zufuhr von Abgas in die Messkammer unterbindet, wenn es die Zufuhr von Nullgas zulässt, und die Zufuhr von Nullgas zulässt, wenn es die Zufuhr von Abgas unterbindet. Dadurch, dass wahlweise ausschließlich Nullgas oder ausschließlich Abgas in die Messkammer einführbar ist, können sowohl der Nullabgleich (nur Nullgas in der Messkammer) als auch die eigentliche Messung (nur Abgas in der Messkammer) besonders effektiv und mit hoher Genauigkeit durchgeführt werden.

Die Nullgasquelle weist wenigstens einen Filter auf.

Durch mit einem Filter gefilterte Umgebungsluft kann geeignetes Nullgas besonders kostengünstig bereitgestellt werden.

In einer Ausführungsform weist die Nullgasquelle einen zweiteiligen Filter auf. Ein zweiteiliger Filter, der insbesondere einen Grobfilter und einen in Strömungsrichtung hinter dem Grobfilter angeordneten Feinfilter aufweist, kann angesaugte Umgebungsluft besonders effektiv filtern. Auch werden die Wartungskosten reduziert, da der Grob- und der Feinfilter bei Bedarf unabhängig voneinander gereinigt bzw. ausgetauscht werden können.

In einer Ausführungsform weist die Nullgasquelle wenigstens einen Drucksensor auf. Durch einen Drucksensor kann der Druck des zugeführten Nullgases überwacht und über eine geeignete Steuervorrichtung geeignet eingestellt werden. Darüber hinaus kann durch die Bestimmung des am Filter auftretenden Druckabfalls des Nullgases der Verschmutzungsgrad des Filters bestimmt werden.

In einer Ausführungsform weist die Nullgasquelle wenigstens eine Pumpe auf. Mit Hilfe einer Pumpe kann das Nullgas besonders effektiv in die Messkammer gefördert werden.

In einer Ausführungsform ist die Pumpe stromabwärts der Messkammer angeordnet, Dadurch kann nach dem Befüllen der Messkammer mit Nullgas durch Absperren der Nullgaszufuhr und Weiterbetreiben der Pumpe ein Unterdruck in der Messkammer erzeugt werden. Da die Konzentration von Partikeln in einem Gas proportional zu dessen Druck ist, kann durch Absenken des Drucks die Partikelkonzentration in der Messkammer unter die Partikelkonzentration im Nullgas abgesenkt werden. Dadurch wird das Nullniveau abgesenkt und die Empfindlichkeit der Messvorrichtung verbessert.

Die Nullgasquelle ist derart mit der Messkammer verbunden, dass aus der Messkammer strömendes Nullgas der Luftzufuhr der Nullgasquelle zugeführt wird. So wird ein geschlossener Kreislauf für das Nullgas geschaffen und das Nullgas wird besonders effektiv genutzt. Dadurch, dass das Nullgas im Kreislauf wiederholt durch den bzw. die Filter geführt wird, kann die Partikelkonzentration im Nullgas noch weiter abgesenkt werden.

In einer Ausführungsform ist das Emissionsmessgerät so eingerichtet, dass der Nullabgleich automatisch durchgeführt wird. Durch einen automatisch durchgeführten Nullabgleich wird sichergestellt, dass das Messgerät unabhängig von den Gepflogenheiten des Benutzers, der einen manuellen Nullabgleich vergessen oder aus Bequemlichkeit auslassen könnte, stets hinreichend genaue Ergebnisse liefert.

Ein automatischer Nullabgleich kann jeweils nach einer vorgegebenen Anzahl von Messungen und/oder in vorgegebenen Zeitintervallen ausgelöst werden. Das Verfahren kann auch so eingerichtet sein, dass ein Nullabgleich automatisch durchgeführt wird, wenn entweder eine vorgegebene Zahl von Messungen erreicht worden ist oder wenn der zeitliche Abstand zwischen zwei aufeinander folgenden Messungen ein vorgegebenes Zeitintervall überschreitet, je nachdem, welches Kriterium zuerst erfüllt ist.

Die Genauigkeit der Nullpunkteinstellung braucht so nur über den Zeitraum zwischen zwei aufeinanderfolgenden Nullabgleichen sichergestellt zu sein. Messfehler aufgrund von Drifterscheinungen können so zuverlässig verhindert bzw. minimiert werden. Im Extremfall kann ein Nullabgleich zwingend und automatisch vor jeder einzelnen Messung durchgeführt werden, um Messergebnisse mit besonders hoher Genauigkeit zu erhalten.

Ausführungsbeispiele der Erfindung werden im Folgenden anhand der beigefügten Figuren beschrieben:
Figur 1 zeigt schematisch eine erfindungsgemäße Messvorrichtung.
Figur 2 zeigt schematisch ein Ausführungsbeispiel einer Messvorrichtung.
Figur 3 zeigt schematisch ein Ausführungsbeispiel einer Messvorrichtung.

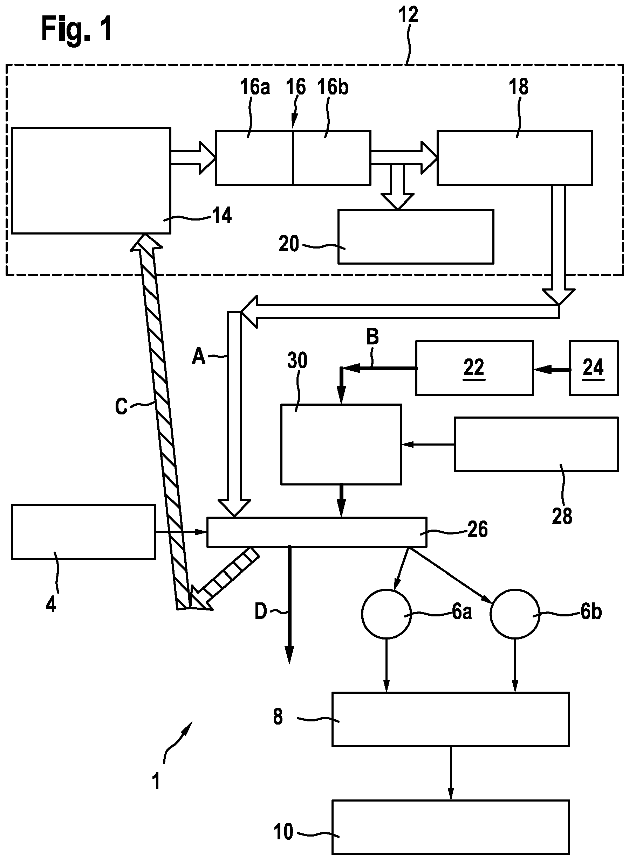

Die in Figur 1 gezeigte Messvorrichtung 1 gemäß der Erfindung weist eine Messkammer 26 mit einer Lichtquelle 4 auf, die z. B. als Laser ausgebildet ist und im Betrieb Licht in die Messkammer 26 strahlt. Die Messkammer 26 ist mit zwei Lichtsensoren 6a, 6b ausgestattet. In der schematischen Darstellung der Figur 1 sind die Lichtsensoren 6a, 6b aus Gründen der besseren Übersichtlichkeit außerhalb der Messkammer 26 dargestellt, obwohl sie in der Realität zumindest teilweise innerhalb oder an der Messkammer 26 angeordnet sind. Die Lichtsensoren 6a, 6b erfassen von der Lichtquelle 4 ausgestrahltes Licht, das von in der Messkammer 26 vorhandenen Partikeln gestreut worden ist (Streulicht). Die Lichtsensoren 6a und 6b sind dabei vorzugsweise derart angeordnet, dass sie jeweils Licht, welches in verschiedenen Winkeln gestreut worden ist, erfassen.

Die beiden Lichtsensoren 6a, 6b sind mit einer Auswerteeinheit 8 verbunden, welche die von den Lichtsensoren 6a, 6b ausgegeben Signale auswertet und dabei insbesondere die Partikelkonzentration in der Messkammer 26 bestimmt. Die Ergebnisse der Auswertung werden über eine Ausgabevorrichtung 10 ausgegeben. Die Ausgabevorrichtung 10 kann eine Anzeigevorrichtung (Display), einen Drucker und/oder eine Datenschnittstelle umfassen, die ausgebildet ist, die Ergebnisse an einen Datenverarbeitungs- oder eine Datenspeichervorrichtung, z.B. eine Diskette oder einen USB-Stick, zu übertragen.

Die zu messenden Abgase eines schematisch dargestellten Kraftfahrzeugs 24 werden von einer Abgassonde 22 aufgenommen, die im oder am Auspuff des Kraftfahrzeugs 24 angebracht ist, und durch einen Abgasschlauch und ein Schaltelement 30 der Messkammer 26 zugeführt (Abgasstrom B). Vor (stromaufwärts) oder hinter (stromabwärts) der Messkammer 26 kann eine in der Figur 1 nicht gezeigte Pumpe vorgesehen sein, um den Abgasstrom zusätzlich zu dem vom Motor des Kraftfahrzeugs 24 erzeugen Abgasdruck zu unterstützen.

Das Schaltelement 30 ist mit einer Steuereinheit 28 verbunden und zwischen einem geöffneten Zustand, in dem es einen Zustrom von Abgasen aus dem Kraftfahrzeug 24 in die Messkammer 26 erlaubt, und einem geschlossenen Zustand, in dem der Zustrom von Abgasen aus dem Kraftfahrzeug 24 in die Messkammer 26 abgeschaltet ist, umschaltbar. Die Steuereinheit 28 ist bspw. elektrisch, mechanisch oder hydraulisch mit dem Schaltelement 30 verbunden, das z. B. als Schaltventil ausgebildet ist.

Aus der Messkammer 26 strömen die Abgase über eine nicht gezeigte Abgasabführvorrichtung nach außen (Abgasstrom D), ohne die unmittelbare Umgebung der Messkammer 26, z. B. die Werkstatt oder den Messplatz, zu verunreinigen oder zu vergiften.

Eine erfindungsgemäße Messvorrichtung 1 weist zusätzlich eine Nullgasquelle 12 auf, die so genanntes Nullgas, d. h., Gas mit einer besonders niedrigen Partikelkonzentration, zur Verfügung stellt. Die Nullgasquelle 12 weist eine Luftzuführung 14 auf, welche Luft aus der Umgebung aufnimmt. Wird die Messvorrichtung 1 in einer besonders verschmutzten und/oder staubhaltigen Umgebung, wie z.B. einer Werkstatt, betrieben, so kann die Luftzuführung 14 mit einem Rohr oder Kamin ausgebildet sein, um die Umgebungsluft aus einer größeren Entfernung, z. B. von außerhalb des Gebäudes heranzuführen. Alternativ kann besonders saubere Luft auch aus angelieferten Gasflaschen entnommen werden.

Die Luftzuführung 14 führt die aufgenommene Umgebungsluft einer Filtereinheit 16 zu, welche dazu ausgebildet ist, die Partikelkonzentration in der aufgenommenen Luft zu reduzieren. Dazu weist die Filtereinheit 16 wenigstens einen Feinfilter 16b (z. B. einen sogenannten HEPA-Filter) auf, der in der Lage ist, die zugeführte Luft so sauber zu filtern, dass die Konzentration der Partikel, die nach der Filterung noch im Nullgas enthalten sind, niedriger als die Partikelkonzentration in Abgasen ist, die von Fahrzeugen mit einem gut funktionierenden Abgas-Partikelfilter abgegeben werden.

Dem Feinfilter 16b ist ein Grobfilter 16a vorgeschaltet, der besonders grobe Partikel aus der zugeführten Luft heraus filtert, bevor sie in den Feinfilter 16b gelangen, und so ein schnelles Verschmutzen und/oder Verstopfen des Feinfilters 16b verhindert. Dadurch können die Wartungsintervalle zum Auswechseln bzw. Reinigen der Filter 16a und 16b verlängert werden. Auch können der Grobfilter 16a und der Feinfilter 16b dem jeweiligen Verschmutzungsgrad entsprechend separat ausgewechselt bzw. gereinigt werden. Durch diese Maßnahmen können die Wartungskosten verringert werden.

Stromabwärts der Filtereinheit 16 ist eine Pumpe 18 vorgesehen, welche Umgebungsluft durch die Luftzuführung 14 und die Filtereinheit 16 ansaugt und an die Messkammer 26 ausgibt (Nullgasstrom A).

Im Verlauf der Luftströmung zwischen der Luftzuführung 14 und der Pumpe 18 ist ein Drucksensor 20 vorgesehen, der den Druck der zugeführten Luft in der Nullgasquelle 12 misst und das Ergebnis an eine in der Figur 1 nicht gezeigte Steuereinheit weitergibt. Der so gemessene Druck kann zur Regelung der Pumpe 18 verwendet werden, um einen ausreichende Nullgasstrom A aus der Nullgasquelle 12 in die Messkammer 26 sicherzustellen.

Bei bekannter Leistung der Pumpe 18 kann aus dem Druck der zugeführten Luft bzw. aus dem an der Filtereinheit 16 auftretenden Druckabfall auf den Verschmutzungsgrad der Filtereinheit 16 geschlossen werden, da über eine verschmutze Filtereinheit 16 ein großer Druckabfall auftritt als über eine saubere Filtereinheit 16. Überschreitet der Druckabfall über die Filtereinheit 16 einen vorgegebenen Grenzwert, so wird ein Warnsignal ausgegeben, welches den Benutzer darauf hinweist, dass wenigstens ein Filter der Filtereinheit 16 auszuwechseln ist. Auch kann die Pumpe 18 bei Überschreiten eines vorgegebenen Grenzwertes, der gleich oder höher als der Grenzwert ist, bei dem eine Warnung ausgegeben wird, abgeschaltet werden, wenn die Verschmutzung der Filtereinheit 16 so stark ist, dass eine sichere Funktion der Nullgasquelle 12 nicht mehr gewährleistet ist. Zusätzlich kann ein zweiter Drucksensor 20 stromaufwärts zwischen der Luftzuführung 14 und der Filtereinheit 16 angeordnet sein.

In dem in der Figur 1 gezeigten 2. Vorrichtung wird das Nullgas, welches aus der Messkammer 26 strömt, erneut der Luftzuführung 14 zugeführt (Nullgasrückstrom C). So entsteht ein Nullgaskreislauf, in dem das Nullgas wiederholt durch die Filtereinheit 16 geführt wird. Dadurch kann die Partikelkonzentration im Nullgas noch weiter reduziert werden. Auch wird der Bedarf an von außen zugeführtem Gas reduziert, was besonders vorteilhaft ist, wenn als Nullgas besonders sauberes aber auch teures Gas aus Gasflaschen verwendet wird.

Vor dem eigentlichen Messvorgang steuert die Steuereinheit 28 das Schaltelement 30 so an, dass keine Abgase von dem Kraftfahrzeug 24 in die Messkammer 26 strömen.

Die Pumpe 18 ist eingeschaltet, so dass durch die Filtereinheit 16 gefiltertes Nullgas aus der Nullgasquelle 12 in die Messkammer 26 strömt.

Durch Einschalten der Lichtquelle 4 und Auswerten der von den Lichtsensoren 6a, 6b ausgegebenen Signale, die auf dem von Partikeln in der Messkammer 26 gestreutem und von den Lichtsensoren 6a, 6b aufgenommenem Licht beruhen, wird ein Nullabgleich der Messkammer 26 durchgeführt.

Nachdem der Nullabgleich durchgeführt worden ist, steuert die Steuereinheit 28 das Schaltelement 30 so an, dass die Zufuhr von Abgasen von dem Kraftfahrzeug 24 in die Messkammer 26 geöffnet ist und Abgase aus dem Kraftfahrzeug 24 durch die Messkammer strömen (Abgasstrom B, C), so dass die Partikelkonzentration in den Abgasen gemessen werden kann. Alternativ kann das Umschalten des Schaltelementes 30 mechanisch durch den Bediener erfolgen. In diesem Fall kann auf einen Motor o.ä. zum Umschalten des Schaltelementes 30 verzichtet werden.

In der in der Figur 1 gezeigten Vorrichtung wird der Nullgasstrom A während der Messung der Partikelkonzentration in den Abgasen nicht abgeschaltet. Das Nullgas aus der Nullgasquelle 12 strömt gleichzeitig mit den zu messenden Abgasen durch die Messkammer 26. Dabei wird das Nullgas beispielsweise als Spülgas unmittelbar vor den Sensoren 6a, 6b und/oder der Lichtaustrittsöffnung der Lichtquelle 4 entlang geführt, um zu verhindern, dass die Sensoren 6a, 6b bzw. die Lichtaustrittsöffnung durch die Ablagerung von Partikeln aus dem Abgasstrom B verschmutzt.

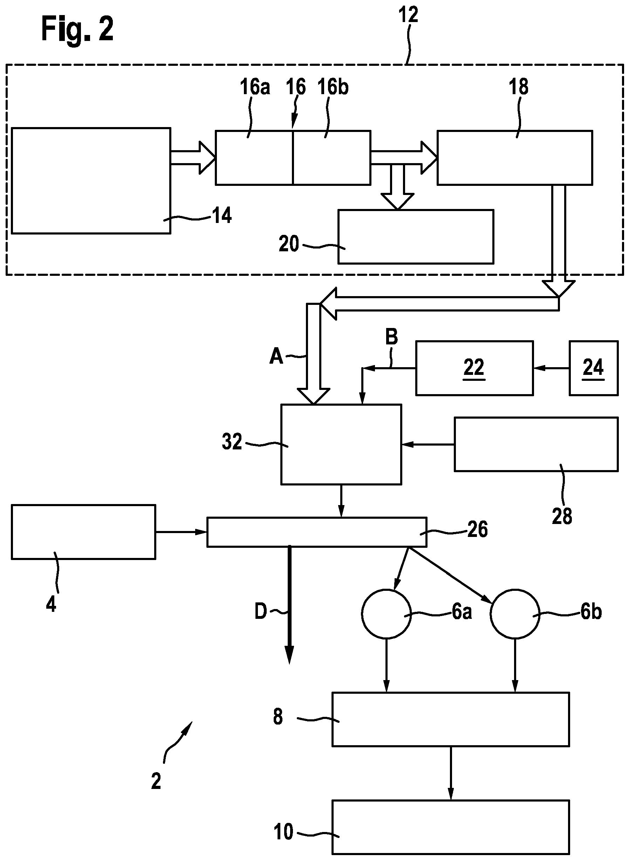

Figur 2 zeigt schematisch ein Ausführungsbeispiel einer Messvorrichtung 2.

Der Aufbau dieser Messvorrichtung 2 entspricht im Wesentlichen dem in der Figur 1 gezeigten Erfindungsbeispiel.

Die gleichen Elemente sind mit den gleichen Bezugszeichen versehen und werden, soweit sie in Aufbau und Funktion mit der ersten Vorrichtung übereinstimmen, nicht erneut beschrieben.

Die Messvorrichtung 2 unterscheidet sich von der Messvorrichtung 1 dadurch, dass das Schaltelement 32 als Umschaltelement ausgebildet ist, so dass die Gaszufuhr in die Messkammer 26 wahlweise zwischen Abgasen von dem Kraftfahrzeug 24 und Nullgas aus der Nullgasquelle 12 umschaltbar ist. D. h., dass während des Messvorgangs, wenn Abgase von dem Kraftfahrzeug 24 in die Messkammer 26 geführt werden, um die Partikelkonzentration in den Abgasen zu bestimmen, kein Nullgas aus der Nullgasquelle 12 in die Messkammer 26 strömt. Ebenso strömt während des Nullabgleichs, wenn das Nullgas aus der Nullgasquelle 12 durch die Messkammer 26 geführt wird, kein Abgas durch die Messkammer 26.

Dadurch, dass während des Messvorgangs kein Nullgas in die Messkammer 26 strömt, wird ein Verdünnen der Abgase des Kraftfahrzeugs 24 in der Messkammer 26 vermieden und auch eine geringe Partikelkonzentration in den Abgasen kann mit hoher Genauigkeit bestimmt werden.

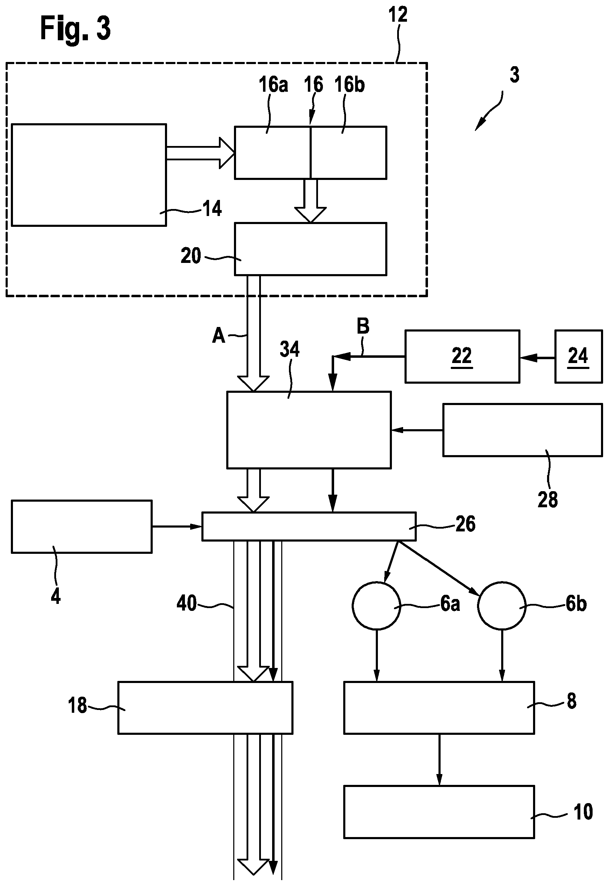

Figur 3 zeigt ein Ausführungsbeispiel einer Messvorrichtung 3.

Auch hier werden die Elemente, die den gleichen Aufbau und die gleiche Funktion wie in den zuvor gezeigten Vorrichtungen haben, nicht erneut beschrieben.

Die Messvorrichtung 3 unterscheidet sich von den zuvor gezeigten Messvorrichtungen 1, 2 dadurch, dass die Pumpe 18, die zur Förderung des Nullgases vorgesehen ist, nicht innerhalb der Nullgasquelle 12 stromaufwärts der Messkammer 26, sondern stromabwärts der Messkammer 26 in einer Abgasleitung 40 angeordnet ist, die vorgesehen ist, um die Abgase aus der Messkammer 26 abzuführen.

In diesem Fall fördert die Pumpe 18 das Nullgas aus der Nullgasquelle 12 durch Ansaugen des Nullgases durch die Messkammer 26. Gleichzeitig unterstützt die Pumpe 18, wenn sie auch während des Messvorgangs betrieben wird, den Abgasstrom B aus dem Kraftfahrzeug 24 durch die Messkammer 26.

In der in der Figur 3 gezeigten Vorrichtung ist das Schaltelement 34 so ausgebildet, dass es anders als im zuvor gezeigten zweiten Ausführungsbeispiel, nicht nur zwischen dem Nullgasstrom A und dem Abgasstrom B umschalten kann, sondern auch eine Einstellung aufweist, in der sowohl die Zufuhr des Abgasstromes B als auch die Zufuhr des Nullgasstromes A abgesperrt sind.

Durch gleichzeitiges Absperren der Zufuhr von Nullgas und Abgasen bei gleichzeitigem Betrieb der Pumpe 18 kann ein Unterdruck in der Messkammer 26 erzeugt werden. Da die Partikelkonzentration in einem Gasvolumen proportional zu dessen Druck ist, kann durch Absenken des Drucks bzw. Erzeugen eines Unterdrucks in der Messkammer 26 die Partikelkonzentration in der Messkammer 26 noch weiter gesenkt werden. So wird ein niedrigeres Nullniveau der Messvorrichtung 3 erreicht und die Erkennungsschwelle für Partikel im Abgasstrom B kann noch weiter abgesenkt werden. Im Ergebnis kann die mit der Messvorrichtung 3 erreichbare Genauigkeit weiter erhöht werden.

Jede der gezeigten Messvorrichtungen 1, 2, 3 kann vorteilhafterweise so ausgebildet sein, dass eine Messung nur dann durchgeführt werden kann, wenn zuvor ein Nullabgleich durchgeführt worden ist. Insbesondere können die Messvorrichtungen 1, 2, 3 so ausgebildet sein, dass ein Nullabgleich automatisch vor jeder Messung vorgenommen wird. Dadurch wird verhindert, dass ein notwendiger Nullabgleich aus Vergesslichkeit oder Bequemlichkeit des Bedieners nicht ausgeführt wird und eine Messung durchgeführt wird, die aufgrund des fehlenden Nullabgleichs falsche Messergebnisse liefert.

Eine Messvorrichtung, welche den Nullabgleich automatisch durchführt, liefert besonders zuverlässig Messergebnisse mit bestmöglicher Genauigkeit.

## Patentansprüche

1. Vorrichtung (1; 2; 3) zur Messung einer Partikelkonzentration in Kraftfahrzeugabgasen mit
- einer Messkammer (26), die mit einer zu messenden Gas-Partikel-Mischung befüllbar ist, wenigstens einer Lichtquelle (4) und wenigstens einem Lichtsensor (6a, 6b), wobei der Lichtsensor (6a, 6b) ausgebildet ist, um von in der Gas-Partikel-Mischung vorhandenen Partikeln gestreutes Licht zu detektieren;
- einer Auswerteeinheit (8), die mit dem wenigstens einen Lichtsensor (6a, 6b) verbunden ist, und die vom Lichtsensor ausgegebenen Signale auswertet;
- einer Abgaszuführeinrichtung (22), die ausgebildet ist, der Messkammer (26) zu messendes Abgas zuzuführen;
- einer Nullgasquelle (12), die ausgebildet ist, ein partikelarmes Nullgas bereitzustellen, wobei die Nullgasquelle (12) eine Filtereinheit (16) und eine Luftzuführung (14) aufweist, welche Luft aus der Umgebung oder aus Gasflaschen aufnimmt und der Filtereinheit (16) zuführt; und
- einem Schaltelement (30; 32; 34), das zwischen der Abgaszuführeinrichtung (22) und der Messkammer (26) angeordnet und ausgebildet ist, die Zufuhr von Abgas in die Messkammer (26) wahlweise zuzulassen oder zu unterbinden,
wobei die Nullgasquelle (12) derart ausgebildet ist, dass ein ausreichender Nullgasstrom A aus der Nullgasquelle (12) in die Messkammer (26) sichergestellt wird, und wobei die Nullgasquelle (12) mit der Messkammer (26) derart verbunden ist, dass Nullgas, welches aus der Messkammer (26) strömt, erneut der Luftzuführung (14) zugeführt wird.

2. Vorrichtung (1; 2; 3) nach einem der vorangehenden Ansprüche, wobei die Filtereinheit (16) wenigstens einen Filter (16a, 16b) aufweist.

3. Vorrichtung (1; 2; 3) nach einem der vorangehenden Ansprüche, wobei die Nullgasquelle (12) wenigstens einen Drucksensor (20) aufweist.

4. Vorrichtung (1; 2) nach einem der vorangehenden Ansprüche, wobei die Vorrichtung (1; 2; 3) wenigstens eine Pumpe (18) aufweist.

5. Vorrichtung (1; 2) nach Anspruch 4, wobei die Pumpe (18) in Strömungsrichtung vor der Messkammer (26) angeordnet ist.

6. Vorrichtung (1; 2; 3) nach einem der vorangehenden Ansprüche, die derart eingerichtet ist, dass automatisch ein Nullabgleich durchgeführt wird.

## Claims

1. Apparatus (1; 2; 3) for measuring a particle concentration in motor vehicle exhaust gases having
- a measuring chamber (26) which can be filled with a gas/particle mixture to be measured, at least one light source (4) and at least one light sensor (6a, 6b), the light sensor (6a, 6b) being configured to detect light which is scattered by particles which are present in the gas/particle mixture;
- an evaluation unit (8) which is connected to the at least one light sensor (6a, 6b) and which evaluates signals which are output by the light sensor;
- an exhaust gas feeding device (22) which is configured to feed exhaust gas to be measured to the measuring chamber (26);
- a zero gas source (12) which is configured to provide a low-particle zero gas, the zero gas source (12) having a filter unit (16) and an air feeding means (14) which receives air from the surroundings or from gas cylinders and feeds it to the filter unit (16); and
- a switching element (30; 32; 34) which is arranged between the exhaust gas feeding device (22) and the measuring chamber (26) and is configured to selectively permit or prevent the feed of exhaust gas into the measuring chamber (26), the zero gas source (12) being configured in such a way that a sufficient zero gas stream A from the zero gas source (12) into the measuring chamber (26) is ensured, and
the zero gas source (12) being connected to the measuring chamber (26) in such a way that zero gas which flows from the measuring chamber (26) is again fed to the air feeding means (14).

2. Apparatus (1; 2; 3) according to one of the preceding claims, the filter unit (16) having at least one filter (16a, 16b).

3. Apparatus (1; 2; 3) according to either of the preceding claims, the zero gas source (12) having at least one pressure sensor (20).

4. Apparatus (1; 2) according to one of the preceding claims, the apparatus (1; 2; 3) having at least one pump (18) .

5. Apparatus (1; 2) according to Claim 4, the pump (18) being arranged upstream of the measuring chamber (26) in the flow direction.

6. Apparatus (1; 2; 3) according to one of the preceding claims which is set up in such a way that a zero adjustment is carried out automatically.

## Revendications

1. Dispositif (1 ; 2 ; 3) servant à mesurer une concentration en particules dans des gaz d'échappement de véhicules automobiles, comprenant
- une chambre de mesure (26) pouvant être remplie d'un mélange gaz-particules à mesurer, au moins une source lumineuse (4) et au moins un capteur de lumière (6a, 6b), dans lequel le capteur de lumière (6a, 6b) est conçu pour détecter la lumière diffusée par des particules présentes dans le mélange gaz-particules ;
- une unité d'analyse (8) qui est reliée à au moins un capteur de lumière (6a, 6b) et analyse les signaux délivrés par le capteur de lumière ;
- un dispositif d'amenée de gaz d'échappement (22) qui est conçu pour amener des gaz d'échappement à mesurer dans la chambre de mesure (26) ;
- une source de gaz zéro (12) qui est conçue pour fournir un gaz zéro pauvre en particules, dans lequel la source de gaz zéro (12) comporte une unité de filtrage (16) et une amenée d'air (14) recevant de l'air de l'environnement ou de bouteilles de gaz et l'envoie à l'unité de filtrage (16) ; et
- un élément de commutation (30 ; 32 ; 34) placé entre le dispositif d'amenée de gaz d'échappement (22) et la chambre de mesure (26) et conçu pour permettre ou empêcher sélectivement l'amenée de gaz d'échappement dans la chambre de mesure (26),
dans lequel la source de gaz zéro (12) est conçue pour assurer un débit de gaz zéro A suffisant de la source de gaz zéro (12) vers la chambre de mesure (26), et dans lequel
la source de gaz zéro (12) est reliée à la chambre de mesure (26) de manière à ce que le gaz zéro qui s'écoule hors de la chambre de mesure (26) soit de renvoyé à l'amenée d'air (14).

2. Dispositif (1 ; 2 ; 3) selon l'une des revendications précédentes, dans lequel l'unité de filtrage (16) comporte au moins un filtre (16a, 16b).

3. Dispositif (1 ; 2 ; 3) selon l'une des revendications précédentes, dans lequel la source de gaz zéro (12) comporte au moins un capteur de pression (20).

4. Dispositif (1 ; 2) selon l'une des revendications précédentes, dans lequel le dispositif (1 ; 2 ; 3) comporte au moins une pompe (18).

5. Dispositif (1 ; 2) selon la revendication 4, dans lequel la pompe (18) est disposée en amont de la chambre de mesure (26) dans la direction d'écoulement.

6. Dispositif (1 ; 2 ; 3) selon l'une des revendications précédentes, qui est conçu de manière à ce qu'un réglage du zéro soit effectué automatiquement.
